# EUROPEAN PATENT SPECIFICATION

(11) **EP 3 773 764 B1**
(45) Date of publication and mention of the grant of the patent: **27.09.2023**
(21) Application number: 19717560.7
(22) Date of filing: 04.04.2019
(51) Int. Cl.: A61L 15/44, A61K 9/00, A61K 47/06, A61K 47/10, A61K 47/12, A61K 47/14, A61K 47/18, A61K 9/06, A61K 47/32, A61K 47/44, A61K 47/46, A61K 31/085, A61L 26/00

(54) **COMPOSITION FOR TREATMENT OF CHRONIC WOUNDS**
ZUSAMMENSETZUNG ZUR BEHANDLUNG VON CHRONISCHEN WUNDEN
COMPOSITION POUR LE TRAITEMENT DE PLAIES CHRONIQUES

(30) Priority: 06.04.2018 GB 201805783
(43) Date of publication of application: 17.02.2021
(73) Proprietor: Pellis Care Limited, Cambridge CB1 3DA (GB)
(72) Inventor: PURVIS, Duncan Ross, Cambridgeshire CB1 3QA (GB); THOMAS, Janette Ann, Cambridgeshire CB1 3QA (GB); BENNETT, Brian, Warwickshire CV11 6JQ (GB); KEELING, Celia, Warwickshire CV9 3LN (GB)
(74) Representative: Stratagem IPM Limited
(86) International application number: PCT/GB2019/050960
(87) International publication number: WO 2019/193333

(56) References cited:
- WO-A2-2006/099359
- GB-A- 2 043 410
- GB-A- 2 403 410
- GLOOR M ET AL: "Triclosan, ein dermatologisches Lokaltherapeutikum", HAUTARZT, SPRINGER VERLAG, BERLIN, DE, vol. 53, 1 November 2002 (2002-11-01), pages 724-729, XP002391035, ISSN: 0017-8470, DOI: 10.1007/S00105-002-0416-Y

## Description

This invention relates to a pharmaceutical composition comprising 2, 4, 4'-trichloro-2'-hydroxydiphenylether (triclosan) for use in the treatment of chronic wounds and in particular for use in the treatment of diabetic foot ulcers.

The management of chronic wounds grade (EPUAP) 3 and 4, a full-thickness skin defect that fails to heal within 3 months - are a major therapeutic challenge which is being exacerbated by the rise of conditions such as diabetes, obesity and vascular disorders that impede wound healing. Up to 70% of chronic wounds can be encouraged to heal within 3 months, but it is very difficult to judge at the outset which will be the 'treatable' wounds; for those stubborn non-healing wounds, there are currently few if any research-driven treatment options.

Despite being clinically and molecularly different, all chronic wounds are generally assigned to one of three major clinical categories: leg ulcers, diabetic foot ulcers or pressure ulcers. There is still much to learn regarding the mechanisms involved in the healing or lack of healing of chronic wound pathology. This could be due to the animal models failing to mirror the clinical features of chronic wounds and as such their use as models further impedes discovery and testing of suitable treatment regimens.

Most chronic wounds are considered to be poorly vascularised. Persistent inflammation is a hallmark of most chronic wounds and they are also infected. This leads to a large influx and retention of innate immune cells into chronic wounds which are likely to inhibit many repair processes. One consistent obstacle in the healing of many chronic wounds is a build-up of necrotic debris at the wound edge. This is the reason it is often clinical practice to debride the wound, to establish a 'fresh new' wound, which can lead to efficient re-epithelialisation.

Pathogenesis of the ulcers is due to neuropathic impairment of musculoskeletal balance, as well as immune compromise from leukocyte dysfunction and peripheral vascular disease, complicating these wounds with infection. Standard of care includes off-loading, attentive debridement, maintenance of a moist wound environment, and, when cellulitis is present, systemic antibiotics. Diabetic Foot Ulcer is an infection, ulceration or destruction of deep tissues associated with neurological abnormalities and various degrees of peripheral vascular diseases in the lower limb (World Health Organization definition, 1995).

Diabetic foot ulcers are common and estimated to affect 15% of all diabetic individuals during their lifetime. Diabetic foot ulcers are the consequence of multiple factors including peripheral neuropathy, decreased blood supply (ischaemia from peripheral arterial disease, and microvascular disease) high plantar pressures and pose a significant risk for morbidity, limb loss and mortality. Peripheral neuropathy has been demonstrated to be the single most common contributing cause of foot ulceration. Ulceration can be attributed to a triad of peripheral neuropathy, biomechanical deformity, and superimposed minor trauma. In general, lack of sensation from neuropathy is responsible for unrecognized repetitive trauma and loading that leads to skin and soft tissue breakdown, creating an entry point for infection. The inflammatory environment contributes to diabetic microangiopathy and worsening local ischemia. Other factors in ulceration are trauma, deformity, callus formation, and oedema. Current treatments for diabetic foot ulcers comprise: Debridement (surgical, enzymatic, and/or by dressing changes) and antibiotics. Debridement removes devitalized tissue, which can be a source of endotoxins that inhibit fibroblast and keratinocyte migration into the wound. In addition to systemic antibiotics and surgical intervention, wound care is an important component of diabetic foot ulcer management.

### Topical Antibiotics in treatment of Ulcers

The lack of available data makes it difficult to assess the efficacy of topical antimicrobials for diabetic foot ulcers. Few systemic agents improve outcomes for diabetic foot ulcers, but several topical substances hasten healing, including silver-containing compounds for venous ulcers and oxyquinoline ointment for stage 1 to 2 pressure ulcers. A Cochrane systematic review (O'Meara et al., Antibiotics and antiseptics for venous leg ulcers. Cochrane Database of Systematic Reviews 2014, Issue 1. Art. No.: CD003557) of antibiotics and antiseptics for venous leg ulcers concluded that some evidence supports the use of cadexomer iodine. The authors also concluded that current evidence does not support the routine use of honey or silver based products and that further evidence is required before conclusions can be drawn about the effectiveness of povidone-iodine, peroxide-based preparations, ethacridine lactate, chloramphenicol, framycetin, mupirocin, ethacridine or chlorhexidine in healing venous leg ulceration. A systematic review (Game et al., Effectiveness of interventions to enhance healing of chronic ulcers of the foot in diabetes: a systematic review. Diabetes/Metabolism Research and Reviews 2016;32(1 Suppl):154-68) of the effectiveness of various interventions for enhancing the healing of chronic diabetic foot ulcers found a single study that demonstrated no benefit of cadexomer-iodine in cavitary wounds and one suggesting that zinc oxide tape improved necrotic wounds more than a hydrocolloid. Another Cochrane review (Bergin et al., Silver based wound dressings and topical agents for treating diabetic foot ulcers. Cochrane Database of Systematic Reviews 2006, Issue 1. Art. No.: CD005082.) of silver-based wound dressings and topical agents for treating diabetic foot ulcers found no randomized controlled trials (RCTs) which reported outcomes on healing rates or infection resolution. Likewise, a Cochrane review (Vermeulen et al., Topical silver for treating infected wounds. Cochrane Database of Systematic Reviews 2007, Issue 1. Art. No.: CD005486) of silver-containing dressings or topical agents for treating infected or contaminated chronic wounds concluded there was insufficient evidence, on the basis of three randomized trials, to recommend these treatments. A Cochrane systematic review (Jull et al., Honey as a topical treatment for wounds. Cochrane Database of Systematic Reviews 2015, Issue 3. Art. No.: CD005083) on topical honey for treating wounds concluded, based on data from 19 trials, that honey may reduce the healing time for mild-to-moderate superficial and partial thickness burns but did not significantly hasten leg ulcer healing. The authors deemed that there was insufficient evidence to guide clinical practice. Finally, a recent systematic review of the effectiveness of interventions in the management of diabetic foot infections found six studies that investigated the use of topical agents, but the methods and results did not allow the authors to draw any definitive conclusions. Among the two studies of topical antibiotics, one found that an antimicrobial peptide, pexiganan cream, was similar in effectiveness to a systemic antibiotic(ofloxacin) in the treatment of mildly infected diabetic foot ulcers, while another study of adjunctive therapy with a gentamicin-collagen sponge (along with systemic antibiotic therapy) was difficult to interpret because of methodological problems (Peters et al., Interventions in the management of infection in the foot in diabetes: a systematic review. Diabetes/Metabolism Research and Reviews 2016; 32(1 Suppl):145-53).

The most frequently used topical antimicrobials in wound care practice are chlorhexidine, iodine, silver containing products, mupriocin and fucidic acid. In the past, acetic acid, honey, hydrogen peroxide, sodium hypochlorite, potassium permanganate, and proflavine have been used.

The basic framework for effective prevention and management of diabetic foot disease often seems to be missing, (National Diabetes Foot Care Audit Report 2014-2016), with no unilateral, effective, treatment method. The traditional treatments currently offer intermit results with many patients experiencing repeat ulcerations and eventually amputation.

A disadvantage with the use of a topical application of antibiotics is few agents have been proven to be effective in clinical trials. In addition, almost all topical applications of antibiotics have minimal penetration into intact skin or soft tissue, which limits use to open wounds without cellulitis or deep soft-tissue spread of infection. Other disadvantages to the use of topical antibiotics include systemic absorption of some agents may occur if used on large wounds; agents may induce local hypersensitivity or contact dermatitis reactions; some agents may interfere with normal wound healing processes; treatment may produce an alteration of normal cutaneous flora that leads to other problems; topical applications are difficult to dose accurately; frequent applications may be needed; agents may be difficult to apply or aesthetically unacceptable to some patients and the stored agent can become contaminated.

Topical antimicrobials have traditionally been formulated in one of two ways. Firstly, ointments, which are more occlusive, often contain petrolatum, and are best used for dry lesions. Secondly, creams, which are less occlusive, wash off with water, are less messy, and are best for moist lesions. One major problem with topical therapies is that no official oversight agency has standardized and approved specific tests to establish the efficacy and safety of these agents. Topical antiseptics are usually avoided in open wounds because they interfere with wound healing because of cytotoxicity to healing cells.

GB 2 403 410 A (Hygieia Pharmaceuticals Limited) discloses triclosan containing pharmaceutical compositions having a viscosity of 20 000 mPa.s or less for use in the treatment of damaged skin. The compositions may be used in the treatment and/or prophylaxis of skin ulcers.

There is a large unmet need for an effective topical treatment for chronic wounds, in particular diabetic foot ulcers and other non-healing ulcerated chronic wounds that result from disease or injury.

### Summary of the invention

The scope of this invention is defined by the claims. Any 'embodiment' or 'example' which is disclosed in the description but is not covered by the claims should be considered as presented for illustrative purposes only. Embodiments in the description relating to methods of treatment are not covered by the claims.

It has been determined that compositions, in accordance with the claims of the present invention, provide an extremely effective and novel clinical solution. Pharmaceutical compositions comprising 2,4,4'-trichloro-2'-hydroxydiphenylether (triclosan); and a thickener at 0.5% - 8% by weight of the composition enables a viscosity between 70000 - 150000 centipoise, or cP (at between 20-40 °C).

1cP corresponds to 1 mPa.s. The specific solution of the invention permits *inter alia* a high and sustained concentration of the antimicrobial agent to be packed and retained in the site of infection, enabling improved effectiveness of the active ingredient without some common disadvantages of the prior art.

Furthermore, the invention facilitates treatment in an outpatient setting and better adherence by the patient to a regime also improves overall treatment effectiveness.

In one embodiment the composition is formulated for topical administration. In particular, in the composition may be in the form of an emulsion but may be in any topical form, such as a cream or paste that is suitable for application, i.e. packing a wound.

Such compositions are shown by the applicant to be particularly effective in the treatment of chronic wounds. In use, the continuous presence of triclosan in the composition of the present invention prevents or reduces broad spectrum microbial growth and inflammatory processes occurring in the underlying wounds. When the active is formulated in the above described way of the present invention it has characteristics which promote ideal conditions for chronic wound healing by being suitable for packing. In a preferred embodiment, the chronic wound is a human wound.

Treatment of a chronic wound may include but is not limited to delay, reduction and/or amelioration of a symptom of the chronic wound, such as swelling, discharge, discoloration, pain, calluses, thickened skin surrounding the wound and in advanced stages fever and chills.

The chronic wound may be any non-healing wound including but not limited to a leg ulcer, a diabetic foot ulcer, a pressure ulcer, a burn or other ulcerating wound from injury or disease.

The chronic wound or ulcer is caused at least in part by one or more of the following: peripheral neuropathy, ischaemia from peripheral arterial disease, microvascular disease, biomechanical abnormalities and superimposed minor trauma.

In particular, use of the present invention for the treatment of diabetic foot ulcers has been shown as particularly effective by the applicant. In many instances, the diabetic foot ulcer has not responded to any previous treatment with systemic or topical antibiotic creams and dressings. The diabetic foot ulcer may typically comprise one or more calluses.

Further, use of the composition for the treatment diabetic foot ulcers is especially advantageous due to the typical requirement of existing treatments to limit or restrict concentration of active ingredient placed at the infection site, as well as reducing potential toxicity risks and systemic absorption.

Treatment of diabetic foot ulcer includes the delay, reduction and/or amelioration of any symptom of diabetic foot ulcer, including, swelling, discharge, discoloration, pain, calluses, thickened skin surrounding the ulcer, in advanced stages fever and chills.

The composition is for use in association with any ulcer which may result from diabetes. The use of the composition may be administered at any time in association with diabetes therapy. For example, the composition may be administered before and/or during and/or after alternative diabetes therapy.

Viscosity is a measure of the resistance of a fluid to deformation under shear stress. It is commonly perceived as "thickness" Dynamic Viscosity can be measured using a viscometer and gives a unit measurement in centipoises (cP) or millipascal seconds (mPa.s). One method is to measure the strain using a Brookfield Viscometer and a T bar C spindle at 10 RPM at room temperature. In some embodiments, the composition may have a viscosity range from 70 000 - 100 000 cP, or 80 000 - 120 000 cP or 100 000 - 130 000 cP or 120 000 - 150 000 cP.

In another embodiment, the thickener may be Carbopol, polyacrylic acid, guar gum, carbomer, cetyl palmitate or other gelling agent.

The composition may further comprise a neutralising agent. The neutralising agent may be selected from one or more of triethanolamine, sodium hydroxide or potassium hydroxide.

The formulation may further comprise water.

The composition may further comprise an emulsifier. The emulsifier may be selected from one or more stearate derivatives, for example, glyceryl monostearate.

In another embodiment, the 2,4,4'-trichloro-2'-hydroxydiphenylether (triclosan) may be present at a concentration of between 0.1 - 2.0% or 0.1-4.0% by weight of the formulation.

In another embodiment, the composition may comprise a silicon based water repellent and/or skin conditioning agent.

In a further embodiment, the composition may comprise absorption promoting adjuvants.

In a yet a further embodiment, the composition may comprise one of more of the following ingredients: a surfactant, a bulking agent, a tonicity adjusting agent, a stabilizer, a preservative and a buffer.

In embodiments the composition further comprises additional elements such as: Vegetable Oils (almond coconut olive), Hydrogenated Castor Oil, Melaleuca Alternifolia (Tea Tree) Leaf Oil, Olea Europaea (Olive) Fruit Oil, Hydrogenated Vegetable Oil, Euphorbia Cerifera (Candelilla) Wax, Glyceryl Dibehenate, Tribehenin, Glyceryl Behenate, L-Lysine HCL, Squalane, Tocopheryl Acetate, Phytosphingosine, Ceramides Castor oil, Stearic acid, Glycerol Stearate, Cetyl Palmitate, Silicone Fluid: Dimethicone, Nipastat, Jojoba Oil, Liquid Paraffin, Carbomer, Triethanolamine, Aloe Vera, Monopropylene Glycol, Tea Tree Oil, Ferulic Acid, Triclocarban, Chlorohexidine (Gluconate or undecylenate), Diclofenac (sodium salt), Hyaluronic acid, Centella Asiatica oil, calendula extract, shea butter, manuka oil.

The composition may comprises at least one hydrophilic component phase; and at least one hydrophobic component phase; wherein either the hydrophobic component phase or the hydrophilic component phase forms the greatest portion of the composition by weight.

In one non-limiting example the composition may comprise the following ingredients in the desirable ranges indicated:

| Ingredient | Range (wt%) |
|---|---|
| Castor oil | 0.5-3.0 |
| Stearic acid | 0.5-8.0 |
| Glycerol mono stearate (GMS SE) | 0.1-3.0 |
| Cetyl palmitate | 0.1-2.0 |
| Silicone fluid 200/100 CS dimethicone | 0.1-10 |
| Additional Preservative | 0.0-0.5 |
| Jojoba oil | 0.1-0.5 |
| Liquid paraffin | 0.1-0.5 |
| 2,4,4'-trichloro-2'-hydroxydiphenylether (Triclosan) | 0.3-10 |
| Water | 35-95 |
| Carbopol 5% | 0.5-8.0 |
| Aloe vera | 0.1-2.0 |
| Monopropylene glycol (MPG) | 2.0-15 |
| Triethanolamine | 0.1-2.0 |

According to some embodiments the composition is formulated for topical administration.

The term "topical" refers to administration of the composition at, or immediately beneath, the point of application. As used herein "topical application" refers to application onto one or more surfaces(s) including keratinous tissue, i.e., "topically applying." Topical application or "topically applying" may involve direct application to the area of the desired substrate. The topical preparation and/or composition may be applied by pouring, dropping, or rubbing on, or by any other appropriate means.

Further, the composition may be in the form of an emulsion, cream, ointment, lotion or balm.

In some embodiments the composition may be combined with further agents such as a therapeutically effective amount of an anti-inflammatory agent and/or an effective amount of an analgesic, to improve patient comfort further.

The composition may be combined with another pharmaceutical agent. In a further embodiment the composition is used in combination with oral, parenteral, or topical medication.

In some embodiments the composition for use in treatment according to the above described applications maybe combined with use of one or more further treatments. These may include debridement treatment, a systemic antibiotic treatment and/or a post-dressing treatment. Debridement may occur immediately before application or packing of the composition into the wound. Antibiotics may be administered concurrently with the treatment of the invention. Dressing the wound may be advantage post treatment, every time the wound is retreated.

In preferred embodiments the treatment comprises packing the wound with the composition, dressing or covering the wound and repeating this treatment at least once, preferably on a regular basis, such as a daily or weekly basis. In some embodiments, especially where the composition has a greater amount of water, the treatment comprises packing the wound with the composition, dressing or covering the wound and repeating this treatment at least twice or three times within a 7 day period. This is to ensure the dressing is not soaked and remains a practical sterile barrier. Such a treatment does not however necessitate debriding of the wound as the excess water acts to flush the wound surface throughout.

The composition can be administered for 3, 4, 5, 6, 7, 8 or more weeks. A peak response is achieved at 8 weeks or more after the treatment commences. The patient can be in remission of symptoms for 4-12, 6-12, 6-18 weeks or more including 16-36 months after treatment.

The actual amount administered, and rate and time-course of administration, will depend on the nature and severity of what is being treated. Prescription of treatment, e.g. decisions on dosage etc, is ultimately within the responsibility and at the discretion of general practitioners and other medical doctors, and typically takes account of the disorder to be treated, the condition of the individual patient, the site of delivery, the method of administration and other factors known to practitioners. The composition may be administered once, twice, three or four times a day or periodically.

The precise dose of the composition will depend upon a number of factors, including the severity of the ulcer. The composition is preferably administered to an individual in a "therapeutically effective amount", this being sufficient to show benefit to the individual.

Also disclosed (not claimed) is a method of manufacturing a pharmaceutical composition according to the claims, wherein the method comprises the steps of:
i) preparing an oil phase comprising the triclosan;
ii) preparing an aqueous phase comprising at least the thickener; and
iii) mixing the oil phase and the aqueous phase together.

In some embodiments, after mixing the phases together one may add further water to adjust the final viscosity of the composition.

Preparation of the aqueous phase may include the addition of one or more of components selected from monopropylene glycol, triethanolamine, water, aloe vera and/or a combination thereof.

Preparation of the oil phase may include one or more components selected from: castor oil, stearic acid, glycerol stearate, cetyl palmitate, silicon fluid, jojoba oil, liquid paraffin and/or a combination thereof.

The invention relates to the composition as previously defined above for use in treating a chronic wound including but not limited to a leg ulcer, diabetic foot ulcer or pressure ulcer in a subject, comprising administering a composition as previously defined above. The composition may be administered as a topical formulation filling the wound, optionally the wound maybe covered with a dressing. In a preferred method the composition is administered before and/or during and/or after debridement and/or systemic antibiotics treatment.

The term "therapeutically effective amount" as used herein in the context of treating diabetic foot ulcers means an amount capable of reducing the size of the ulcer of the subject before the composition of the invention is administered.

Treatment may include curative, alleviation or prophylactic effects. The term 'treatment' is used herein to refer to any regimen that can benefit a human.

More specifically, treatment includes "therapeutic" and "prophylactic" and these types of treatment are to be considered in their broadest context. The term "therapeutic" does not necessarily imply that a subject is treated until total recovery. Similarly, "prophylactic" does not necessarily mean that the subject will not eventually contract a disease condition.

Accordingly, therapeutic and prophylactic treatment includes amelioration of the symptoms of a particular condition or preventing or otherwise reducing the risk of developing a particular condition. The term "prophylactic" may be considered as reducing the severity or the onset of a particular condition. "Prophylactic" also includes preventing reoccurrence of a particular condition in a patient previously diagnosed with the condition. "Therapeutic" may also reduce the severity of an existing condition.

The invention will now be further described by way of reference to the following Examples and Patient treatments.

These are provided for the purposes of illustration only and are not to be construed as being limiting on the invention.

### Figures

Figure 1 (a) is a picture of a diabetic foot ulcer in patient 1. The first ulcer is on the dorsal interphalangeal joint on the second toe, size of 5p, 1cm sq;
Figure 1(b) is a further picture of patient 1 and the second diabetic foot ulcer is on the apex 1^{st} toe, before debridement;
Figure 2 (a) is a further picture, after debridement;
Figure 2 (b) is a further picture after 3-4 weeks;
Figure 3 (a) is a further picture after 4-6 weeks;
Figure 3 (b) is a further picture at 6 weeks;
Figure 4 (a) is a further picture at 8-9 weeks;
Figure 4 (b) is a further picture just before it was fully healed;
Figure 5 (a) (b) is a further picture of the healed apex of 1^{st} toe;
Figure 6a is a picture of a diabetic foot ulcer of patient 2, the picture shows the ulcer before (left) and after (right) debridement and cleaning;
Figure 6b is a further picture of the diabetic foot ulcer of patient 2, taken in 4/5-week intervals from date of first treatment; and
Figure 7 is a further picture of patient 2, when the ulcer is fully healed.

### Examples

The formulations shown below were prepared as follows:
1. The castor oil, stearic acid, glycerol stearate, cetyl palmitate, silicon fluid, jojoba oil and liquid paraffin were melted together.
2. Mixed together well and heated to 60 °C.
3. In a separate vessel the monopropylene glycol, triethanolamine, 50% of water and carbomer pH 5.5 was mixed together using a Silverson High Shear Mixer 20 000 rpm fine mesh head.
4. The mixture was heated to 65 °C
5. The active component (triclosan) was added to the mixture from step (1) immediately before stage (6) and mixed.
6. The oil phase mixture from step (1) was added to the water phase from step (3) and mixed well under high shear conditions using a Silverson High Shear Mixer.
7. When fully mixed aloe vera solution was added & stirred in.
8. The remaining cold water was added and stirred in. Final pH 5.5
9. The mixture was left overnight to stand and cool, then re-mixed the total batch.

The mixture was poured/filled while still warm at about 40°C.

Component wt % range in composition examples:

| Ingredient | Range (wt. %) |
|---|---|
| Castor oil | 0.5-3.0 |
| Stearic acid | 0.5-8.0 |
| Glycerol mono stearate (GMS SE) | 0.1-3.0 |
| Cetyl palmitate | 0.1-2.0 |
| Silicone fluid 200/100 CS dimethicone | 0.1-10 |
| Additional Preservative | 0.0-0.5 |
| Jojoba oil | 0.1-0.5 |
| Liquid paraffin | 0.1-0.5 |
| Triclosan | 0.3-10 |
| Water | 35-95 |
| Carbopol 5% | 0.5-8.0 |
| Aloe vera | 0.1-2.0 |
| Monopropylene glycol (MPG) | 2.0-15 |
| Triethanolamine | 0.1-2.0 |

By varying thickener ratio of 0.5 % to up to 8 % of the composition weight, as per the examples below, the viscosity of the resulting product was between 70 000-150 000 cP. The paste was typically thickened using Carbopol Triethanolamine, however one could use polyacrylic acids, Guar gum or Xanthan Gum as the thickener or other standard cosmetic or pharmaceutical thickeners suitable for topical use.

A number of different examples compositions were then produced with the following wt %:

### EXAMPLE 1

| Materials | Wt. % | Phase |
|---|---|---|
| Castor oil | 2.00 | |
| Stearic acid | 6.00 | A |
| GMS SE | 2.00 | |
| Cetyl Palmitate | 1.00 | |
| Silicone fluid | 1.00 | |
| Additional Preservative | 0.20 | |
| Jojoba oil | 0.10 | |
| Liquid paraffin | 0.10 | |
| MPG | 10.00 | B |
| Triethanolamine | 1.55 | |
| Hot water @ 65°C | 39.65 | |
| Carbopol | 5.00 | |
| Triclosan | 0.50 | C |
| Aloe vera | 0.50 | D |
| Fragrance | 0.15 | |
| Cold water | 30.25 | E |

The ingredients of phase A were melted together at a temperature of 60 °C. The ingredients of phase B were mixed together using a Silverson High Shear Mixer. Phase C was added to phase A and stirred until dissolved. Phase B was then added to phase A. Phase D was then added and mixed using a Silverson High Shear Mixer. Phase E was also added while mixing.

### EXAMPLE 2:

| Materials | Wt. % | Phase |
|---|---|---|
| Castor oil | 2.0 | |
| Stearic acid | 1.5 | |
| GMS SE | 0.5 | |
| Cetyl Palmitate | 0.5 | |
| Silicone fluid | 1.0 | |
| Additional Preservative | 0.2 | A |
| Jojoba oil | 0.1 | |
| Liquid paraffin | 0.1 | |
| Triclosan | 1.0 | B |
| Hot water @ 60°C | 80.4 | C |
| Carbopol (5%) | 2.0 | |
| Aloe vera 10.1 | 0.5 | |
| MPG | 10.0 | |
| Triethanolamine | 0.2 | D |

The ingredients of phase A were melted together at a temperature of 70 °C until the phase was clear. The ingredients of phase C were mixed together using a Silverson High Shear Mixer. Phase B was added to phase A and stirred until dissolved. Phase C was then added to phase A. Phase D was then added and mixed using a Silverson High Shear Mixer.

### EXAMPLE 3

| Materials | Wt. % | Phase |
|---|---|---|
| Castor oil | 2.0 | |
| Stearic acid | 1.5 | |
| GMS SE | 0.5 | |
| Cetyl Palmitate | 0.5 | |
| Silicone fluid | 1.0 | |
| Additional Preservative | 0.2 | A |
| Jojoba oil | 0.1 | |
| Liquid paraffin | 0.1 | |
| Triclosan | 2.0 | B |
| MPG | 10.0 | C |
| Hot water @ 60°C | 79.4 | |
| Carbopol | 2.0 | |
| Aloe vera | 0.5 | |
| Triethanolamine | 0.2 | D |

The ingredients of phase A were melted together at temperature 65 °C until clear. The ingredients of phase C were mixed together using a Silverson High Shear Mixer. Phase B was added to phase A and stirred until dissolved. Phase C was then added to phase A. Phase D was then added and mixed using a Silverson High Shear Mixer.

### EXAMPLE 4

| Materials | Wt. % | Phase |
|---|---|---|
| Castor oil | 2.0 | |
| Stearic acid | 1.5 | |
| GMS SE | 0.5 | |
| Cetyl Palmitate | 0.5 | |
| Silicone fluid | 1.0 | |
| Additional Preservative | 0.2 | A |
| Jojoba oil | 0.1 | |
| Liquid paraffin | 0.1 | |
| Triclosan | 2.0 | B |
| MPG | 10.0 | C |
| Hot water @ 60°C | 78.6 | |
| Carbopol 5% | 2.0 | |
| Aloe vera 10:1 | 0.5 | |
| Triethanolamine | 1.0 | D |

The ingredients of phase A (oil) were melted together at a temperature 65-80 °C until clear. The ingredients of phase C (aq) were mixed together using a Silverson High Shear Mixer. Phase B was added to phase A (oil) and stirred until dissolved. Phase C (aq) was then added to phase A (oil). Phase D was then added and mixed using a Silverson High Shear Mixer.

### EXAMPLE 5

| Materials | Wt. % | Phase |
|---|---|---|
| Castor Oil | 2 | A |
| Stearic Acid | 1.5 | |
| G.M.S SE | 0.5 | |
| Cetyl Palmitate | 0.5 | |
| Silicone Fluid 200 100CS | 1 | |
| Jojoba Oil | 0.1 | |
| Liquid Paraffin Heavy | 0.1 | |
| Nipastat (optional preservative) | 0-0.2 | |
| Triclosan | 2 | B |
| Water Hot (60°C) | 32 | C |
| Carbopol 980 5% Sol | 2 | |
| Mono Propylene Glycol | 10 | |
| Triethanolamine | 1 | |
| Aloe Vera 10:1 | 0.5 | |
| Water Cold | 46.6 | D |

Example 5 was prepared as outlined in example 4.

### CLINICAL USE EXAMPLES

A triclosan cream formulation, according to an embodiment of the invention (Example 5), was applied to a number of patients, with differently presented conditions and symptoms. as described below:

### Patient Profile 1:

40 year female diabetic. Neuro ischaemic. Heart rate: pulse mono and biphasic depending on the weather. Indication of reduced arterial blood flow due to a blockage or low elasticity. Ex-smoker. Stage 3 Non healing dorsal diabetic foot ulcer.

The patient had 2 ulcers:
1) one on the dorsal IPJ on the 2nd toe, size of 5 pence, 1 cm sq and
2) one on apex of 1st toe, size of 5p, 1 cm sq.

No osteomyelitis was apparent in the patient.

### Previous Treatment:

Prescribed Flucloxacilin antibiotic for 6 months intermittently and ongoing, this treatment was discontinued two weeks into the treatment with the triclosan cream. After 6 treatment courses the patient could not clear the infection in the wound.

Also treated patient with silver and iodine products and separately honey but no healing occurred.

Toe became sausage-like. With her co-morbidities and weight, a toe amputation would not be advised as she may not survive surgery.

### Treatment with Triclosan cream:

The patient was treated twice a week. The ulcer was filled with the cream to the brim, so the cream was level with skin surface. The cream was not washed off between treatments, but the ulcer had a hard debridement to base of ulcer before each treatment. Triclosan cream was applied for 8 weeks. The cream was packed into the wound and a non-adhesive foam dressing was applied. The patient then wore rocker bottom soles to reduce the weight and pressure. Treatment for this toe ulcer started on 3/11/17 and healed on 20/1/18. No other medication was used.

### Results:

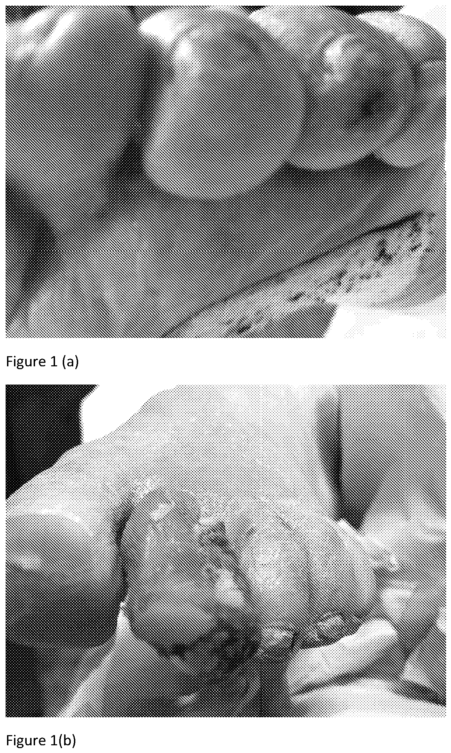

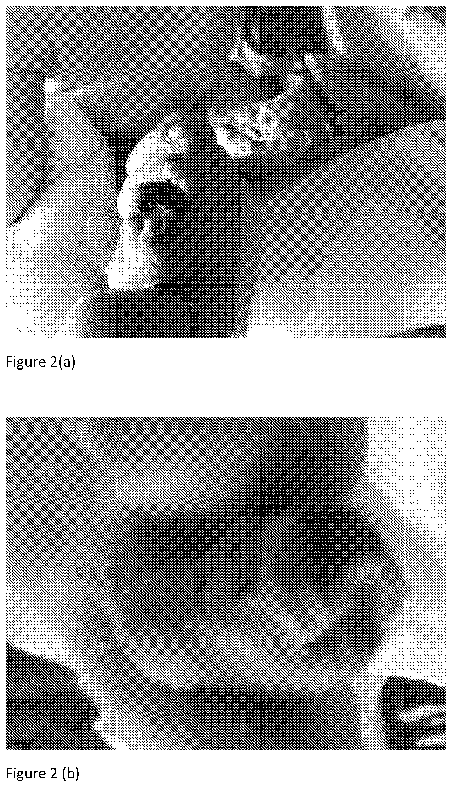

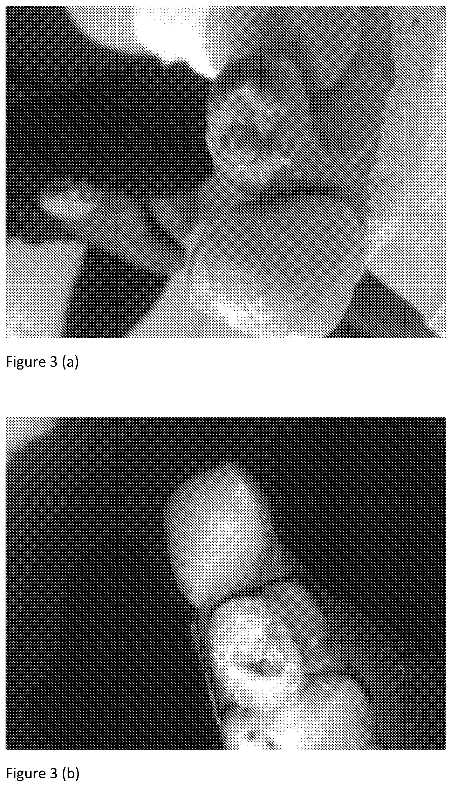

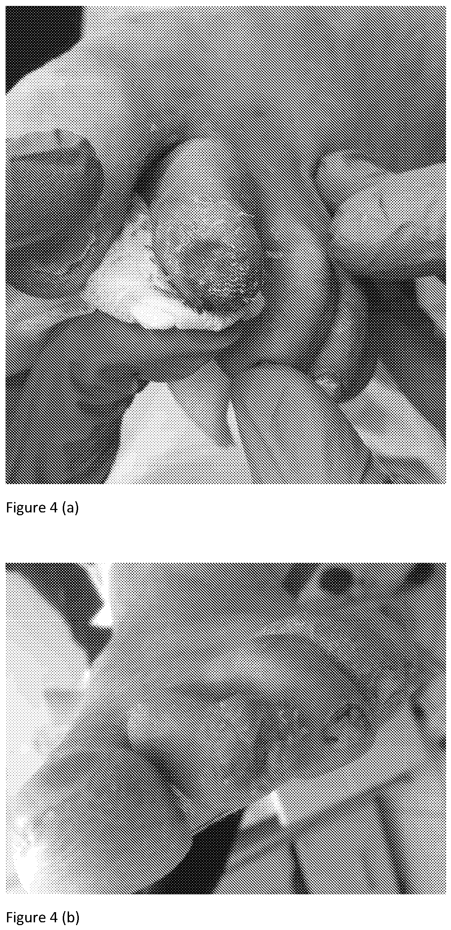

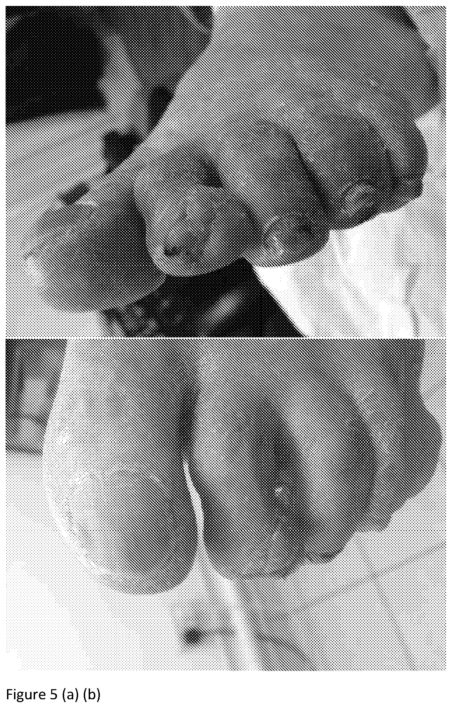

Both ulcers healed fully. Figures 1 to 5 demonstrate further triclosan cream of the invention is effective at treating diabetic foot ulcers.

### Patient Profile 2:

The male patient had 1 ulcer on sole of foot, approximately the size of 50 pence.

No osteomyelitis was apparent in the patient.

### Previous Treatment:

After treatment course of Flucloxacilin antibiotics the patient could not clear the infection in the wound. Also treated patient with silver and iodine products and separately honey but no healing occurred. Chemotherapy treatment. Debridement performed on ulcer and cleaning.

### Treatment with Triclosan cream:

While the patient was receiving chemotherapy, the triclosan cream was applied for 8 weeks. The triclosan cream was applied every alternate day, with a sharp debridement and cleansed with sterile saline solution prior to application of the cream. In the last two weeks of treatment the cream was applied daily. The cream was packed into the ulcer so as to be level with the skin surface and a non-adhesive dressing was applied. The patient then wore rocker bottom soles to reduce the weight and pressure.

### Results:

Ulcer fully healed.

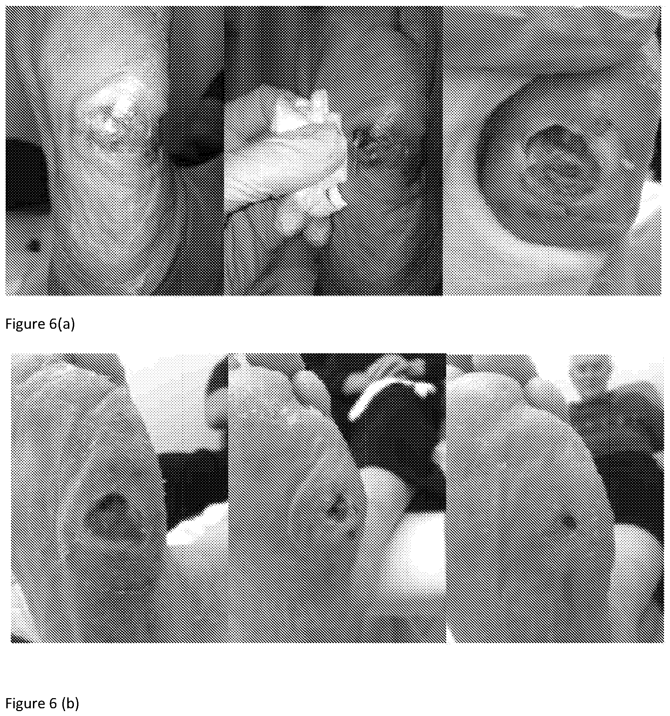

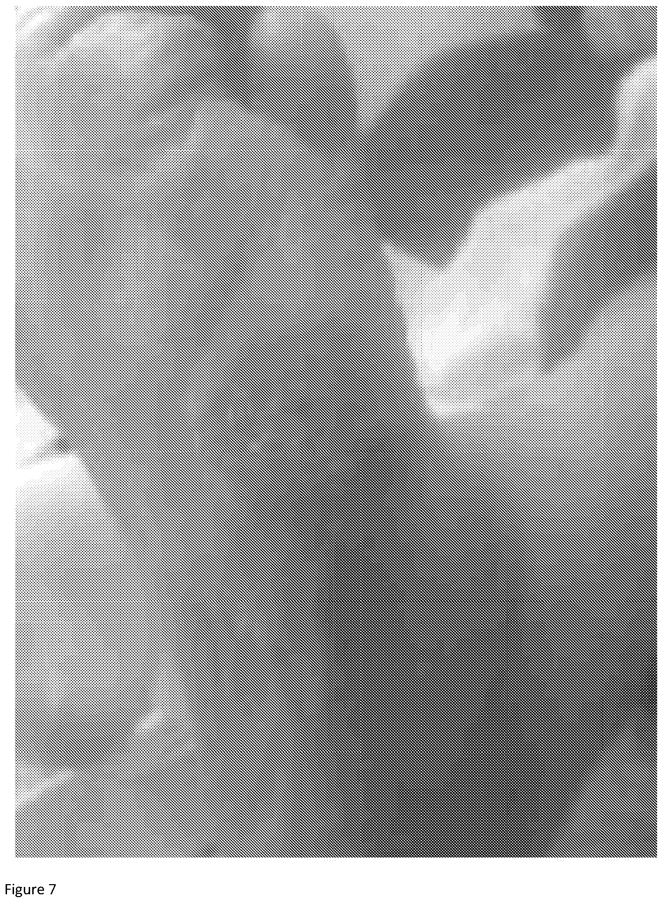

Figures 6 and 7 demonstrate triclosan cream of the invention is effective at treating diabetic foot ulcers.

### Patient Profile 3:

Female, 65 years old, with Reynaud's Disease. The patient had very poor circulation with a monophasic pulse in feet. The patient was suffering with severe chilblains that had started to break down and ulcerate forming a chronic wound.

### Previous Treatment:

No other treatment was helping to heal the ulcerations.

### Treatment with Triclosan cream:

The cream was applied into the wound and dressed with a Biatane dressing and then re-packed and re-dressed once a week. After one month the skin had healed and a further 2 weeks for the chilblain to resolve.

### Patient Profile 4:

Female, 92 years old, ischaemic limbs, wound trauma to the top of her foot. The wound was filling with fluid and not healing.

### Previous Treatment:

Wound care nurses had used standard of care (dressing) to treat her for over 2 months with no improvement.

### Treatment with Triclosan cream:

The cream was applied (packed into the wound) and dressed with a Biatane dressing and then re-packed and re-dressed once a week. The wound healed in 3 weeks.

### Patient Profile 5:

Female, 50 years old, kidney removed 5 years prior. Verrucae on the bottom of the foot. Patient tried to treat herself with bazooka (verruca treatment) gel and nitrous oxide gas. She then went to doctors with cellulitis and a non-healing hole in her foot.

### Previous Treatment:

lodflex was used for a week, but the patient became very poorly with infection and was on verge of needing IV antibiotics.

### Treatment with Triclosan cream:

The cream was packed into the hole, dressed with a Biatane dressing and then re-packed and re-dressed once a week. The patient to felt better within a couple of days and the wound healed after 3-4 weeks of treatment advantageously removing the need for IV antibiotics.

## Claims

1. A pharmaceutical composition for use in treating a chronic wound, the pharmaceutical composition comprising:
2,4,4'-trichloro-2'-hydroxydiphenylether (triclosan); and
at least one thickener at 0.5 - 8 % by weight of the composition, wherein the composition has a viscosity between 70 000 - 150 000 mPa.s (centipoise (cP)) at between 20-40 °C, wherein the chronic wound is defined as a full thickness skin defect that fails to heal within 3 months.

2. A pharmaceutical composition for use according to claim 1, formulated for topical administration.

3. A pharmaceutical composition for use according to claim 1 or 2, wherein the composition is in the form of an emulsion, cream, ointment, lotion or balm.

4. A pharmaceutical composition for use according to any preceding claim, wherein the thickener is selected from one or more of carbopol, polyacrylic acids, guar gum, carbomer, cetyl palmitate and/or other gelling agent.

5. A pharmaceutical composition for use according to any preceding claim, wherein the 2,4,4'-trichloro-2'-hydroxydiphenylether is present at 0.1 - 4.0 % by weight of the composition, more preferably at 0.1 - 2.0 % by weight of the composition.

6. A pharmaceutical composition for use according to any preceding claim, wherein the composition further comprises a therapeutically effective amount of an anti-inflammatory agent and/or an effective amount of an analgesic.

7. A pharmaceutical composition for use according to any preceding claim, wherein the composition comprises at least one hydrophilic component; and at least one hydrophobic component; wherein either the hydrophobic component or the hydrophilic component forms the greatest portion of the composition by weight.

8. A pharmaceutical composition for use according to any preceding claim, wherein the chronic wound is selected from a non-healing wound including, but not limited to, a leg ulcer, a diabetic foot ulcer, a pressure ulcer, a burn or other ulcerating wound from injury or disease.

9. A pharmaceutical composition for use according to any preceding claim, wherein the chronic wound is caused at least in part by one or more of the following: peripheral neuropathy, ischaemia from peripheral arterial disease, microvascular disease, biomechanical abnormalities and superimposed minor trauma.

10. A pharmaceutical composition for use according to any preceding claim, combined with use of one or further treatments selected from a debridement treatment, a systemic antibiotic treatment and/or a post-dressing treatment.

11. A pharmaceutical composition for use according to any preceding claim, wherein the treatment comprises packing the wound with the composition, dressing or covering the wound and repeating the treatment at least once, preferably on a regular basis.

## Patentansprüche

1. Pharmazeutische Zusammensetzung zur Verwendung bei der Behandlung einer chronischen Wunde, wobei die pharmazeutische Zusammensetzung umfasst:
2,4,4'-Trichlor-2'-hydroxydiphenylether (Triclosan); und
mindestens ein Verdickungsmittel mit 0,5-8 Gew.-% der Zusammensetzung, wobei die Zusammensetzung eine Viskosität zwischen 70.000-150.000 mPa.s (Centipoise (cP)) bei zwischen 20-40 °C aufweist, wobei die chronische Wunde als Defekt der Vollhaut definiert ist, der innerhalb von 3 Monaten nicht heilt.

2. Pharmazeutische Zusammensetzung zur Verwendung nach Anspruch 1, formuliert für die topische Verabreichung.

3. Pharmazeutische Zusammensetzung zur Verwendung nach Anspruch 1 oder 2, wobei die Zusammensetzung in Form einer Emulsion, Creme, Salbe, Lotion oder eines Balsams vorliegt.

4. Pharmazeutische Zusammensetzung zur Verwendung nach einem der vorhergehenden Ansprüche, wobei das Verdickungsmittel aus einem oder mehreren von Carbopol, Polyacrylsäuren, Guarkernmehl, Carbomer, Cetylpalmitat und/oder einem anderen Geliermittel ausgewählt ist.

5. Pharmazeutische Zusammensetzung zur Verwendung nach einem der vorhergehenden Ansprüche, wobei der 2,4,4'-Trichlor-2'-hydroxydiphenylether in einer Menge von 0,1 bis 4,0 Gew.-% der Zusammensetzung, bevorzugter in einer Menge von 0,1 bis 2,0 Gew.-% der Zusammensetzung vorhanden ist.

6. Pharmazeutische Zusammensetzung zur Verwendung nach einem der vorhergehenden Ansprüche, wobei die Zusammensetzung ferner eine therapeutisch wirksame Menge eines entzündungshemmenden Mittels und/oder eine wirksame Menge eines Analgetikums umfasst.

7. Pharmazeutische Zusammensetzung zur Verwendung nach einem der vorhergehenden Ansprüche, wobei die Zusammensetzung mindestens eine hydrophile Komponente umfasst; und mindestens eine hydrophobe Komponente; wobei entweder die hydrophobe Komponente oder die hydrophile Komponente den größten Gewichtsanteil der Zusammensetzung ausmacht.

8. Pharmazeutische Zusammensetzung zur Verwendung nach einem der vorhergehenden Ansprüche, wobei die chronische Wunde ausgewählt ist aus einer nicht heilenden Wunde, einschließlich, aber nicht beschränkt auf, einem Beingeschwür, einem diabetischen Fußgeschwür, einem Druckgeschwür, einer Verbrennung oder einer anderen ulzerierenden Wunde aus Verletzung oder Krankheit.

9. Pharmazeutische Zusammensetzung zur Verwendung nach einem der vorhergehenden Ansprüche, wobei die chronische Wunde zumindest teilweise durch eine oder mehrere der folgenden verursacht wird: periphere Neuropathie, Ischämie aufgrund einer peripheren arteriellen Erkrankung, mikrovaskuläre Erkrankung, biomechanische Abnormitäten und überlagertes leichtes Trauma.

10. Pharmazeutische Zusammensetzung zur Verwendung nach einem der vorhergehenden Ansprüche, kombiniert mit der Anwendung einer oder weiterer Behandlungen, die aus einer Debridement-Behandlung, einer systemischen Antibiotika-Behandlung und/oder einer Nachbehandlung ausgewählt werden.

11. Pharmazeutische Zusammensetzung zur Verwendung nach einem der vorhergehenden Ansprüche, wobei die Behandlung das Packen der Wunde mit der Zusammensetzung, das Verbinden oder Bedecken der Wunde und die mindestens einmalige, vorzugsweise regelmäßige Wiederholung der Behandlung umfasst.

## Revendications

1. Composition pharmaceutique destinée à être utilisée dans le traitement d'une plaie chronique, la composition pharmaceutique comprenant :
du 2,4,4'-trichloro-2'-hydroxydiphényléther (triclosan) ; et
au moins un épaississant à raison de 0,5 à 8 % en poids de la composition, la composition comportant une viscosité comprise entre 70 000 et 150 000 mPa.s (centipoises (cP)) entre 20 et 40 °C, ladite plaie chronique étant définie comme étant un défaut cutané de pleine épaisseur qui ne parvient pas à guérir dans un délai de 3 mois.

2. Composition pharmaceutique destinée à être utilisée selon la revendication 1, formulée pour une administration topique.

3. Composition pharmaceutique destinée à être utilisée selon la revendication 1 ou 2, ladite composition se présentant sous la forme d'une émulsion, d'une crème, d'une pommade, d'une lotion ou d'un baume.

4. Composition pharmaceutique destinée à être utilisée selon l'une quelconque des revendications précédentes, ledit épaississant étant choisi parmi un ou plusieurs composants parmi le carbopol, les acides polyacryliques, la gomme de guar, le carbomère, le palmitate de cétyle et/ou un autre agent gélifiant.

5. Composition pharmaceutique destinée à être utilisée selon l'une quelconque des revendications précédentes, ledit 2,4,4'-trichloro-2'-hydroxydiphényléther étant présent à raison de 0,1 à 4,0 % en poids de la composition, plus préférablement à raison de 0,1 à 2,0 % en poids de la composition.

6. Composition pharmaceutique destinée à être utilisée selon l'une quelconque des revendications précédentes, ladite composition comprenant en outre une quantité thérapeutiquement efficace d'un agent anti-inflammatoire et/ou une quantité efficace d'un analgésique.

7. Composition pharmaceutique destinée à être utilisée selon l'une quelconque des revendications précédentes, ladite composition comprenant au moins un composant hydrophile ; et au moins un composant hydrophobe ; soit le composant hydrophobe soit le composant hydrophile formant la plus grande partie de la composition en poids.

8. Composition pharmaceutique destinée à être utilisée selon l'une quelconque des revendications précédentes, ladite plaie chronique étant choisie parmi une plaie non cicatrisante comprenant, sans caractère limitatif, un ulcère de jambe, un ulcère du pied diabétique, une escarre, une brûlure ou une autre plaie ulcéreuse issue d'une blessure ou d'une maladie.

9. Composition pharmaceutique destinée à être utilisée selon l'une quelconque des revendications précédentes, ladite plaie chronique étant causée au moins en partie par un ou plusieurs des éléments suivants : une neuropathie périphérique, une ischémie issue d'une maladie artérielle périphérique, une maladie microvasculaire, des anomalies biomécaniques et un traumatisme mineur superposé.

10. Composition pharmaceutique destinée à être utilisée selon l'une quelconque des revendications précédentes, associée à l'utilisation d'un ou plusieurs traitements choisis parmi un traitement de débridement, un traitement antibiotique systémique et/ou un traitement post-pansement.

11. Composition pharmaceutique destinée à être utilisée selon l'une quelconque des revendications précédentes, ledit traitement comprenant l'application de la composition sur la plaie, le pansage ou le recouvrement de la plaie et la répétition du traitement au moins une fois, de préférence sur une base régulière.
